(19) Europäisches Patentamt
European Patent Office
Office européen des brevets

(11) Publication number: 0 126 743 B1

# (12) EUROPEAN PATENT SPECIFICATION

(45) Date of publication of patent specification: 12.04.89

(51) Int. Cl.$^4$: A 01 N 1/00

(21) Application number: 83903752.0

(22) Date of filing: 03.11.83

(86) International application number:
PCT/US83/01703

(87) International publication number:
WO 84/01879 24.05.84 Gazette 84/13

## (54) SURFACTANT TREATMENT OF IMPLANTABLE BIOLOGICAL TISSUE TO INHIBIT CALCIFICATION.

(30) Priority: 12.11.82 US 441023

(43) Date of publication of application:
05.12.84 Bulletin 84/49

(45) Publication of the grant of the patent:
12.04.89 Bulletin 89/15

(84) Designated Contracting States:
CH DE FR GB LI

(56) References cited:
US-A-3 318 774
US-A-4 323 358
US-A-4 402 697
US-A-4 405 327

(73) Proprietor: BAXTER INTERNATIONAL INC. (a Delaware corporation)
One Baxter Parkway
Deerfield Illinois 60015 (US)

(72) Inventor: NASHEF, Aws, S.
944 West Victoria
Costa Mesa, CA 92627 (US)
Inventor: AHMED, Ahmed, I.
813 Navajo Drive
Riverside, CA 92507 (US)

(74) Representative: Seaborn, George Stephen et al
c/o Edward Evans & Co. Chancery House 53-64
Chancery Lane
London WC2A 1SD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 126 743 B1

## Description

With the introduction of glutaraldehyde preservation of biological tissue, and in particular porcine bioprosthetic heart valves, it has become possible to: a) overcome the poor performance of early formaldehyde-preserved implanted tissue valves; b) discontinue the use of homograft valves; and c) avoid the undesirable use of anticoagulants required to prevent thromboembolism associated with the use of non-bioprosthetic (mechanical) heart valves, especially in children. Not unlike other similarly important discoveries, however it appears that the glutaraldehyde-preserved bioprosthesis has created its own dilemma.

Although the relatively biologically inert glutaraldehyde-preserved valves of Carpentier and others have demonstrated excellent long-term durability in most instances, serious drawbacks such as tissue-fatigue and a propensity toward calcification have occurred. Moreover, it was initially contemplated that children and adolescents would be among those deriving the greatest benefit from the glutaraldehyde-preserved bioprosthetic heart valves since the anticoagulants required with mechanical prosthesis could be eliminated. Results from an increasing number of recent clinical studies indicate that severe calcification of these tissues with relatively short-term failure is prevalent among children and adolescents. Thus, despite their long-term durability and overall reduced incidence of complications, these glutaraldehyde-preserved valves have beem deemed by some to be unsuitable for use in children.

Calcification of tissue remains a mystery for the most part; however, it has previously been shown that various factors including calcium metabolism diseases, age, diet, degeneration of tissue components such as collagen, and turbulance are all involved to a certain extent. Recently, the occurrence of a specific calcium-binding amino acid (gamma carboxyglutamic acid), laid down after implantation of glutaraldehyde-preserved porcine xeno-grafts, has been demonstrated; and it has been postulated to play a role in calcification. While calcification has been accompanied by degradative changes in the glutaraldehyde-treated collagen fibers of the implanted tissue, it remains unclear whether the dystrophic calcification is a cause or the result of tissue degeneration. Nevertheless, there has been a continued effort to elucidate the source of the calcification problem with implanted tissue.

In US—A—4 323 358 mitigation of calcification of porcine heart valve prosthetic devices was brought about by treating the tissue with a sulfated higher aliphatic alcohol such as sodium dodecyl sulphate but no mention was made of preventing post-implantation calcification using other anionic surfactants. Also in EP—A—0 103 946, which was published after the priority date of the present invention, implantable tissue including porcine heart valve was treated with a particular quaternary ammonium salt, dodecyl trimethyl ammonium chloride in order to prevent calcification of the said tissue. No further possibilities were explored.

In accordance with the present invention, we have developed a process which effectively reduces calcification of implanted biological tissue, and maintains the proper hemodynamic properties of the valve leaflets in bioprosthetic heart valves. This process advantageously reduces the tendency of bioprostheses toward calcification and overcomes some of the problems associated with the durability of xenograft heart valves.

In accordance with the present invention, disclosed is an improved process for treating biological tissue prior to implantation which results in a mitigation or reduction of calcification thereof after implantation. The process comprises contacting the biological tissue with a surfactant in an amount effective in reducing calcification of said tissue after implantation.

In accordance with the present invention, it is contemplated that various types of implantable biological tissue derived from numerous animal sources and parts of the anatomy can be made resistant to calcification. Thus, the tissue can be derived from various sources such as but not limited to bovine, porcine, horse, sheep, kangaroo, or rabbit; and can include tendons, ligaments, heart valves, or tissue used to construct heart valves such as dura meter and pericardium. It is further contemplated that tissue used for augmentation such as skin patches, pericardial patches, aortic patches, and tympanic membranes is suitable in the present invention. In accordance with a preferred embodiment of the present invention, porcine heart valves or pericardial tissue which was fixed in glutaraldehyde and subsequently treated with a surfactant was implanted subcutaneously in rabbits. This treated tissue unexpectedly and beneficially effected a sustained mitigation or reduction of calcification after implantation. This sustained mitigation of calcification provides a method of increasing the durability of implanted tissue, particularly of heart valve bioprostheses.

In accordance with the present invention, the tissue may be stored and processed in conventional well-known conditions and may be fixed (tanned) conventionally in from 0.2 to about 0.6 weight percent and preferably from about 0.5 to 0.7 weight percent glutaraldehyde in either phosphate-buffered solutions, or phosphate-free buffers as described hereinafter. The tissue handling conditions as conventionally known are not considered part of our present invention unless otherwise stated. Likewise, tissue may be sterilized in 0.625 percent glutaraldehyde or from 4 to 5 percent formaldehyde.

Organic surfactants within the scope of the present invention include anionic, cationic, and nonionic surfactants. Anionic surfactants of the present invention are ethylene oxide sulfates of aliphatic alcohols, sulfates of alkylphenols, alkane

sulfonic acids, alkylaryl sulfonic acids, bile acids, fatty acids, N-alkanoyl amino acids, N-acylated amino acids, sulfated ethanol amines, and sulfonated alkylphenyl ethers. Such anionic surfactants include those having a relatively large hydrophobic region of hydrocarbon residues including both aliphatic groups, aromatic groups and combinations thereof bonded to a negatively charged ionic group. The aliphatic residues may be branched chains, straight chains, cyclic, heterocyclic, saturated or unsaturated. These hydrophobic residues may either be connected directly to an anionic group such as carboxylate, sulfate, or sulfonate; or connected thereto through an intermediate linkage such as an ester, amide, sulfonamide, ether, or aryl group. Anionic surfactants in one embodiment of the present invention are those having carboxylates bonded to the alkyl side chain of a steroid or through amino acids in the side chain; such as in the bile acids in accordance with the present invention include but are not limited to deoxycholic acid, cholic acid, lithocholic acid, taurocholic acid, and glycocholic acid, and their salts. A preferred bile acid and its salt which we have found effective in mitigation of calcification of implanted tissue is sodium deoxycholate. Anionic surfactants in accordance with the present invention further include those having a carboxylate group bonded to a straight-chained aliphatic group preferably having from 8 to 20 carbon atoms; such as the sodium salts of fatty acids. Anionic surfactants containing carboxylate groups in accordance with the present invention further include those having the carboxylate group coupled to a hydrophobic portion through an amide, sulfonamide, or ester linkage such as in the N-alkanoyl amino acids and N-acylated amino acids. Illustrative of N-alkanoyl amino acids are those including but not limited to surfactants having the formula $R_1CONR_2CHR_3CO_2$— where $R_1$ is an aliphatic residue preferably having from 8 to 18 carbon atoms, $R_2$ is hydrogen or methyl, and $R_3$ is a conventional amino acid side chain. Illustrative side chains include the non-polar aliphatic side chains of alanine, leucine, isoleucine, valine, and proline; the aromatic rings of phenylalanine and tryptophan; the polar side chains of glycine, serine, threonine, cystine, and the like; and the charged polar groups of aspartic acid, glutamic acid, and lysine. Preferred carboxylate containing surfactants in accordance with this embodiment of the present invention are those containing an amide linkage such as N-lauroylsarcosine.

Anionic surfactants in accordance with the present invention also include ethylene oxide modified sulfates of aliphatic alcohols, sulfated ethanol amides and alkyl phenols such as the sulfonated alkylphenyl ethers. Further anionic surfactants include alkane sulfonic acids and alkylaryl sulfonic acids. Alkane sulfonic acids in accordance with the present invention include those having the sulfur directly attached to the hydrophobic residue, such as 1-decanesulfonic acid; or coupled through an ester, amide, or ether; such as N-methyltaurine. Alkylaryl sulfonates are those having the sulfur directly attached to an aromatic ring such as phenyl or naphthyl which is, in turn, coupled to the hydrophobic residue preferably having from 8 to 18 carbon atoms. Illustrative of this latter type of surfactant is dodecylbenzenesulfonic acid.

Cationic surfactants in accordance with the present invention are selected from halide salts of trimethylphenylamine and hexadecyltrimethylamine. Such salts may be chloride or bromide salts. Illustrative surfactants include trimethylphenylammonium chloride and hexadecyltrimethylammonium bromide.

Nonionic surfactants in accordance with the present invention include polyoxyalkylene ethers, polyoxyalkylene alkylaryl ethers, aliphatic esters, polyethers, polyoxyalkylene ester derivatives, saccharide ester derivatives, and combinations thereof. Nonionic polyoxyalkylene, and preferably polyoxyethylene, ethers are those having a relatively long hydrophobic residue and a hydroxyl end connected by one or more alkylene oxide residues. Examples of polyoxyalkylene ethers are polyoxyethylene lauryl ether (Brij), polyoxyethylene oleyl ether and polyoxyethylene cetyl ether. Nonionic polyoxyalkylene, and preferably polyoxyethylene, alkylaryl ethers are those having a relatively large hydrophobic residue and a hydroxyl end connected thereto by an aryl, such as benzene or naphthaline and one or more alkylene oxide residues. Examples of polyoxyalkylene alkylaryl ethers include polyethylene Glycol p-Isooctyl phenyl ethers such as Triton X-100® and the like. Nonionic polyethers include that having the formula

$$CH_3(CH_2)_N—O—(C_2H_4O)_M$$

where N is 11, and M is 23.

Nonionic aliphatic esters include aliphatic fatty acid esters, polypropyleneglycol fatty acid esters such as propyleneglycol monostearate, and glycerol fatty acid esters such as glycerol monostearate. Aliphatic fatty acid esters are those having the formula $R_4COOR_5$ where $R_4$ is an alkyl preferably having from 8 to 20 carbon atoms, and $R_5$ is an aliphatic residue having from 1 to 5 carbon atoms. Saccharide and polyoxyalkylene ester derivatives are those having either a 5 or 6 carbon sugar in the former or a polyoxyalkylene chain in the latter coupled to a relatively long hydrophobic residue through an ester bond. Illustrative saccharide derivatives include sorbitol coupled to fatty acids to form surfactants such as sorbitan trioleate, sorbitan stearate and sorbitan monooleate. Polyoxyalkylene ester derivatives include polyoxyethylene monooleate, polyoxyethylene monostearate, and the like. Combinations of polyoxyalkylene ether derivatives and sorbitol ester derivatives found to be useful in the present invention include polyoxyethylene sorbitan fatty acid derivatives such as polyoxyethylene (20) sorbitan monooleate (Polysorbate-80®, Tween-80® manufactured by DIFCO).

In accordance with the present invention, the effective concentration of surfactant will vary somewhat depending on the molecular weight thereof; and is preferably from 0.1 to 10 percent (w/v) and more preferably from 0.5 to 5 percent. Most preferably, the surfactant concentration is from 0.5 to 1.5 percent. Moreover, the treatment of the tissue with surfactant can be performed during the fixation (tanning) process, during the sterilization process, or in a separate step after fixation and prior to sterilization; and from 2 to 30 hours and preferably from 6 to 24 hours.

In accordance with a preferred embodiment of the present invention, the tissue is treated with surfactant at temperatures of from 20°C to 40°C. In one embodiment, the surfactant is included in the sterilization step whose effectiveness has been found to be enhanced at temperatures above room temperature (20°C) to a range of from 30 to 40°C.

In accordance with the present invention, it is preferable to store, fix, and sterilize the tissue within a tissue-stabilizing pH range; that is, within a pH range that is not deleterious to the tissue components. A preferred pH range is from 7.0 to 7.6, and a more preferred pH range is from 7.1 to 7.4. The most preferred pH in accordance with the present invention is 7.3.

Buffers used in accordance with one embodiment of the present invention are preferably stable, non-interacting with the stabilization process, and have a buffering capacity sufficient to maintain an acceptable pH, particularly during the fixation of the tissue. The choice of the appropriate buffer, and its concentration will depend upon specific tissue preparation conditions; variations of which have been introduced by several manufacturers. The buffers can be either conventional 0.01—0.02 M phosphate-buffered saline (PBS) or phosphate-deficient solutions such as those containing less phosphate than these 0.01 to 0.02 M PBS solutions, and preferably less than 0.001 to 0.002 M phosphate. Preferred buffers in accordance with the present invention include borate, carbonate, bicarbonate, cacodylate (found to be non-toxic in animals), and other synthetic, artifical, or organic buffers such as HEPES N - 2 - hydroxyethylpiperazine - N' - 2 - ethanesulphonic acid; MOPS, 2 - (N - morpholino) propane - sulfonic acid; and PIPES, 1,4 - piperazinediethanesulphonic acid.

Preferably, the buffered or unbuffered solutions, used in accordance with the present invention should not interfere with the tissue stabilizing process afforded by fixing agents such as glutaraldehyde. That is, they should not react with the fixing agent or prevent the fixing agent from achieving proper fixation of the tissue. Illustrative of this are buffers containing primary and secondary amines such as tri(hydroxymethyl)aminomethane (Tris), which are known to react with the aldehyde groups of glutaraldehyde or formaldehyde and thus interfere with the normal tissue stabilization process.

The present invention is further illustrated by the following examples which are not intended to be limiting:

Example I

Extracted porcine aortic heart valve tissue was thoroughly rinsed and shipped in an isotonic (285+15 milliosmols) solution of 0.02M phosphate-buffered saline (0.885 weight percent sodium chloride) at pH 7.3 and at about 4°C; and fixed in 0.625 weight percent glutaraldehyde in an isotonic phosphate-buffered solution at pH 7.4 and at room temperature.

Example II

Extracted porcine aortic heart valve tissue was thoroughly rinsed and shipped in an isotonic (285+15 milliosmols) solution containing 0.54 grams/liter of the sodium salt of N - 2 - hydroxyethylpiperazine - N' - 2 - ethanesulphonic acid (HEPES) and 0.885 weight percent sodium chloride at pH 7.3 at about 4°C; and fixed with 0.625 weight percent glutaraldehyde in an isotonic solution containing 5.39 grams/liter of the sodium salt of HEPES (0.02M), 0.440 weight percent sodium chloride and 2.6 grams/liter of $MgCl_2 \cdot 6H_2O$ at room temperature.

Example III

The extracted tissue of Example I was further sterilized in a 0.02M phosphate-buffered saline (0.885 weight percent sodium chloride) solution (3 square inches of tissue in 70 ml) containing 4+0.4 percent sorbitan monooleate polyoxyethylene (Tween-80®), pH 7.3 at 35°C. The tissue was removed from the solution after 24 hours, rinsed 4 times with 0.625 percent glutaraldehyde in 0.02M phosphate-buffered saline for 10 minutes each and implanted subcutaneously in growing rabbits. The valve tissue was retrieved up to six weeks later at regular one-week intervals. After retrieval, the extent of tissue calcification was assessed by quantitatively monitoring the weight percent calcium in dried tissue using atomic absorption analysis; and histologically by visually monitoring the degree of calcification in Von Kossa-stained tissue sections. Both the histological and quantitative results indicate that the implanted valve tissue effected a significant reduction in calcification compared to a valve tissue treated identically to that described herein in all essential details with the exception that no Tween-80® was added.

Example IV

The extracted tissue of Example II was further sterilized in a 0.02M HEPES (5.39 gram/liter of the sodium salt) buffered saline solution (19.35 square centimetres (3 square inches) of tissue in 70 ml) containing 4+0.4 percent formaldehyde, 22.5 percent ethanol, 0.26 grams/liter $MgCl_2 \cdot 6H_2O$, at pH 7.3 and 35°C. The tissue was removed from the solution after 24 hours, rinsed 4 times with 0.625 percent glutaraldehyde in 0.02M HEPES-buffered saline for 10 minutes each, and implanted subcutaneously in growing

rabbits. The valve tissue was retrieved up to six weeks later at regular one-week intervals. After retrieval, the extent of tissue calcification was assessed by quantitatively monitoring the weight percent calcium in dried tissue using atomic absorption analysis; and histologically by visually monitoring the degree of calcification in Von Kossa-stained tissue sections. The results of the histological and quantitative analyses were used for comparison with results obtained for tissue treated with various surfactants.

Example V

The extracted tissue of Example II was further sterilized in a 0.02M HEPES (5.39 gram/liter of the sodium salt) buffered saline solution (19.35 square centimetres 3 square inches of tissue in 70 ml) containing 4+0.4 percent formaldehyde, 22.5 percent ethanol, 11.3 mM (1.5 weight percent) sorbitan monooleate polyoxyethylene (Tween-80)®, 0.26 grams/liter $MgCl_2 \cdot 6H_2O$, at pH 7.3 and 35°C. The tissue was removed from the solution after 24 hours, rinsed 4 times with 0.625 percent glutaraldehyde in 0.02M HEPES-buffered saline for 10 minutes each, and implanted subcutaneously in growing rabbits. The valve tissue was retrieved up to six weeks later at regular one-week intervals. After retrieval, the extent of tissue calcification was assessed by quantitatively monitoring the weight percent calcium in dried tissue using atomic absorption analysis; and histologically by visually monitoring the degree of calcification in Von Kossa-stained tissue sections. Both the histologic and quantitative results indicate that the implanted valve tissue effected a significant reduction in calcification compared to the valve tissue treated in accordance with Example IV which did not include surfactant.

Example VI

The extracted tissue of Example II was treated identically to that of Example V in all essential details with the exception that no ethanol was added. After sterilization and rinsing the tissue was implanted in growing rabbits and retrieved up to six weeks later at regular one-week intervals. After retrieval, the extent of tissue calcification was assessed by quantitatively monitoring the weight percent calcium in dried tissue using atomic absorption analysis; and histologically by visually monitoring the degree of calcification in Von Kossa-stained tissue sections. Both the histologic and quantitative results indicate that there was no effect of the presence of ethanol in the surfactant solution on mitigating calcification.

Example VII

The extracted tissue of Example II was treated, implanted in growing rabbits, and analyzed identically to that of Example V in all essential details with the exception that 24.0 mM Triton X-100® (1.5 weight percent) was used in place of Tween-80®. The results indicate that the implanted valve tissue effected a significant reduction in calcification compared to the valve tissue treated in accordance with Example IV which did not include surfactant.

Example VIII

The extracted tissue of Example II was treated, implanted in growing rabbits, and analyzed identically to that of Example V in all essential details with the exception that 57.2 mM 1-decanesulfonic acid (1.5 weight percent) was used in place of Tween-80®. The results indicate that the implanted valve tissue effected a significant reduction in calcification compared to the valve tissue treated in accordance with Example IV which did not include surfactant.

Example IX

The extracted tissue of Example II was treated, implanted in growing rabbits, and analyzed identically to that of Example V in all essential details with the exception that 45.9 mM dodecylbenzenesulfonic acid (1.5 weight percent) was used in place of Tween-80®. The results indicate that the implanted valve tissue effected a significant reduction in calcification compared to the valve tissue treated in accordance with Example IV which did not include surfactant.

Example X

The extracted tissue of Example II was treated, implanted in growing rabbits, and analyzed identically to that of Example V in all essential details with the exception that 42 mM potassium coconut fatty acid hydrolyzed protein (Maypon-4C) (1.5 weight percent) was used in place of Tween-80®. The results indicate that the implanted valve tissue effected a significant reduction in calcification compared to the valve tissue treated in accordance with Example IV which did not include surfactant.

Example XI

The extracted tissue of Example II was treated, implanted in growing rabbits, and analyzed identically to that of Example V in all essential details with the exception that 55.3 mM N-lauroylsarcosine (1.5 weight percent) was used in place of Tween-80®. The results indicate that the implanted valve tissue effected a significant reduction in calcification compared to the valve tissue treated in accordance with Example IV which did not include surfactant.

Example XII

The extracted tissue of Example II was treated, implanted in growing rabbits, and analyzed identically to that of Example V in all essential details with the exception that 36.2 mM deoxycholic acid (1.5 weight percent) was used in place of Tween-80®. The results indicate that the implanted valve tissue effected a significant reduction in calcification compared to the valve tissue treated in accordance with Example IV which did not include surfactant.

Example XIII

The extracted tissue of Example II was treated, implanted in growing rabbits, and analyzed identically to that of Example V in all essential details with the exception that 41.2 hexadecyltrimethylammonium bromide (1.5 weight percent) was used in place of Tween-80®. The results indicate that the implanted valve tissue effected a significant reduction in calcification compared to the valve tissue treated in accordance with Example IV which did not include surfactant.

Example XIV

The extracted tissue of Example II was treated, implanted in growing rabbits, and analyzed identically to that of Example V in all essential details with the exception that 87.4 trimethylphenylammonium chloride (1.5 weight percent) was used in place of Tween-80®. The results indicate that the implanted valve tissue effected a significant reduction in calcification compared to the valve tissue treated in accordance with Example IV which did not include surfactant.

Example XV

The tissue treated in accordance with the process of Example III was further analyzed to assess the integrity of the tissue after exposure to surfactant. The results of our analysis show that there was no significant difference in the cross-link stability as indicated by shrinkage temperature, tissue stability as indicated by pronase digestion; amino acid analysis, ninhydrin analysis; uronic acid content, histologic examination as indicated by staining with Hematoxylin-Eosin, aldehyde, fuchsin, PAS/alcian blue, and Trichrome; and surface morphology as determined by scanning electron microscopy and transmission electron microscopy.

**Claims**

1. A process for treating biological tissue prior to implantation in an animal to inhibit calcification comprising the steps of:

fixing the biological tissue under tissue fixing conditions; and

contacting the fixed biological tissue with a solution of a nonionic surfactant in an amount effective in reducing calcification of the tissue after implantation.

2. The process of Claim 1, wherein the surfactant is selected from polyoxyalkylene ethers, polyoxyalkylene alkylaryl ethers, polyoxyalkylene esters, sorbitan esters, and polyoxyalkylene sorbitan esters.

3. The process of Claim 1 or 2, wherein the tissue is contacted with surfactant during fixation.

4. The process of Claim 1 or 2, wherein fixed tissue is contacted with surfactant during post-fixation sterilization.

5. The process of any preceding Claim, wherein the amount of surfactant in the solution is from 0.1 to 10 weight percent.

6. The process of Claim 5, wherein the amount of surfactant in the solution is from 0.5 to 5 weight percent.

7. The process of Claim 5 or 6, wherein the tissue is contacted with the surfactant solution for a time from 2 to 30 hours.

8. The process of Claim 7, wherein the surfactant is sorbitan monooleate polyoxyethylene.

9. The process of any preceding Claim, wherein the tissue is fixed with glutaraldehyde.

10. The process of Claim 1, wherein the tissue is fixed with glutaraldehye; and is contacted with a solution having from 0.1 to 10 weight percent of surfactant selected from polyoxyalkylene ethers, polyoxyalkylene alkylaryl ethers, polyoxyalkylene esters, sorbitan esters, and polyoxyalkylene sorbitan esters.

11. The process of any preceding Claim, wherein the biological tissue is tendon, ligament, heart valve, dura mater, or pericardium.

12. The process of Claim 1, wherein the amount of surfactant in the solution is from 0.1 to 1.5 weight percent, the biological tissue is tendon, ligament, heart valve, dura mater, or pericardium, and is fixed with glutaraldehyde; the surfactant is sorbitan monooleate polyoxyethylene; and the tissue is contacted with the surfactant solution for a time from 6 to about 24 hours at a pH of from 7.0 to 7.6.

13. The process of Claim 12, wherein the tissue is contacted with surfactant during post-fixation sterilization and the surfactant solution further comprises from 4 to 5 percent formaldehyde.

14. A process for treating biological tissue prior to implantation in an animal to inhibit calcification comprising the steps of:

fixing the biological tissue under tissue fixing conditions; and

contacting the fixed biological tissue with a solution of a cationic surfactant in an amount effective in reducing calcification of the tissue after implantation, wherein the cationic surfactant is selected from halide salts of trimethylphenylamine and hexadecyltrimethylamine.

15. The process of Claim 14, wherein the surfactant is selected from trimethylphenylammonium chloride and hexadecyltrimethylammonium bromide.

16. The process of Claim 14, wherein the salt is chloride or bromide.

17. The process of any of Claims 14 to 16, wherein the tissue is contacted with the surfactant during fixation.

18. The process of any of Claims 14 to 16, wherein the tissue is contacted with the surfactant during post-fixation sterilization.

19. The process of Claim 14, wherein the amount of surfactant in the solution is from 0.1 to 10 weight percent.

20. The process of Claim 19, wherein the amount of surfactant in the solution is from 0.5 to 5 weight percent.

21. The process of Claim 19 or 20, wherein the tissue is contacted with the surfactant solution for a time from 2 to 30 hours.

22. The process of any of Claims 14 to 21,

wherein the tissue is fixed with glutaraldehyde.

23. The process of Claim 14, wherein the tissue is fixed with glutaraldehyde; and is contacted with a solution having from 0.1 to 10 weight percent of surfactant selected from trimethylphenylammonium halide and hexadecyltrimethylammonium halide.

24. The process of any of Claims 14 to 23, wherein the biological tissue is tendon, ligament, heart valve, dura mater, or pericardium.

25. The process of Claim 14, wherein the amount of surfactant in the solution is from 0.1 to 1.5 weight percent; the biological tissue is tendon, ligament, heart valve, dura mater, or pericardium, and is fixed with glutaraldehyde; the surfactant is selected from trimethylphenylammonium halide, and hexadecyltrimethylammonium halide; and the tissue is contacted with the surfactant solution for a time from 6 to 24 hours at a pH of from 7.0 to 7.6.

26. The process of Claim 25, wherein the tissue is contacted with surfactant during post-fixation sterilization and the surfactant solution further comprises from 4 to 5 percent formaldehyde.

27. A process for treating biological tissue prior to implantation in an animal to inhibit calcification comprising the steps of:

fixing the biological tissue under tissue fixing conditions; and

contacting the fixed tissue with a solution with the salt of an anionic surfactant in an amount effect in reducing calcification of the tissue after implantation; wherein the surfactant is selected from ethylene oxide sulfates of aliphatic alcohols, sulfates of alkylphenols, alkane sulfonic acids, alkylaryl sulfonic acids, bile acids, fatty acids, N-alkanoyl amino acids, N-acylated amino acids, sulfated ethanol amides, and sulfonated alkylphenyl ethers.

28. The process of Claim 27, wherein the surfactant is the sodium, potassium or ammonium salt.

29. The process of Claim 27 or 28, wherein the tissue is contacted with the surfactant during fixation.

30. The process of Claim 27 or 28, wherein the tissue is contacted with the surfactant during post-fixation sterilization.

31. The process of any of Claims 27 to 30, wherein the amount of surfactant in the solution is from 0.1 to 10 weight percent.

32. The process of Claim 31, wherein the amount of surfactant in the solution is from 0.5 to 5 weight percent.

33. The process of Claim 31 or 32, wherein the tissue is contacted with the surfactant solution for a time from 2 to 30 hours.

34. The process of any of Claims 27 to 33, wherein the tissue is fixed with glutaraldehyde.

35. The process of Claim 27, wherein the tissue is fixed with glutaraldehyde; and is contacted with a solution having from 0.1 to 10 weight percent of surfactant selected from ethylene oxide sulfates of aliphatic alcohols, sulfates of alkylphenols, alkane sulfonic acids and alkylaryl sulfonic acids.

36. The process of any of Claims 27 to 35, wherein the biological tissue is tendon, ligament, heart valve, dura mater or pericardium.

37. The process of Claim 27, wherein the amount of surfactant in the solution is from 0.1 to 1.5 weight percent; the biological tissue is tendon, ligament, heart valve, dura mater or pericardium, and is fixed with glutaraldehyde; the surfactant is N-lauroylsarcosine; and the tissue is contacted with the surfactant solution for a time from 6 to 24 hours at a pH of from 7.0 to 7.6.

38. The process of Claim 37, wherein the tissue contacted with surfactant during post-fixation sterilization and the surfactant solution further comprises from 4 to 5 percent formaldehyde.

**Patentansprüche**

1. Verfahren zum Behandeln von biologischem Gewebe vor der Implantation in ein Tier, um Calcifikation zu hemmen, umfassend folgende Schritte:

Fixieren des biologischen Gewebes unter Gewebefixierbedingungen; und

Kontaktieren des fixierten biologischen Gewebes mit einer Lösung eines nichtionischen Tensids in einer zur Hemmung von Calcifikation des Gewebes nach der Implantation wirksamen Menge.

2. Verfahren nach Anspruch 1, wobei das Tensid ein Polyoxyalkylenether, Polyoxyalkylenalkarylether, Polyoxyalkylenester, Sorbitanester oder Polyoxyalkylensorbitanester ist.

3. Verfahren nach Anspruch 1 oder 2, wobei das Gewebe während der Fixierung mit Tensid kontaktiert wird.

4. Verfahren nach Anspruch 1 oder 2, wobei fixiertes Gewebe während der auf die Fixierung folgenden Sterilisation mit Tensid kontaktiert wird.

5. Verfahren nach einem der vorhergehenden Ansprüche, wobei die Tensidmenge in der Lösung, 0,1—10 Gew.-% beträgt.

6. Verfahren nach Anspruch 5, wobei die Tensidmenge in der Lösung 0,5—5 Gew.-% beträgt.

7. Verfahren nach Anspruch 5 oder 6, wobei das Gewebe mit der Tensidlösung über einen Zeitraum von 2—30 h kontaktiert wird.

8. Verfahren nach Anspruch 7, wobei das Tensid Sorbitanmonooleat - Polyoxyethylen ist.

9. Verfahren nach einem der vorhergehenden Ansprüche, wobei das Gewebe mit Glutaraldehyd fixiert wird.

10. Verfahren nach Anspruch 1, wobei das Gewebe mit Glutaraldehyd fixiert und mit einer Lösung kontaktiert wird, die 0,1—10 Gew.-% Tensid enthält, das ein Polyoxyalkylenether, Polyoxyalkylenalkylarylether, Polyoxyalkylenester, Sorbitanester oder Polyoxyalkylensorbitanester ist.

11. Verfahren nach einem der vorhergehenden Ansprüche, wobei das biologische Gewebe Sehne, Ligament, Herzklappe, Dura mater oder Herzbeutel ist.

12. Verfahren nach Anspruch 1, wobei die Tensidmenge in der Lösung 0,1—1,5 Gew.-%

beträgt, das biologische Gewebe Sehne, Ligament, Herzklappe, Dura mater oder Herzbeutel und mit Glutaraldehyd fixiert ist; das Tensid Sorbitanmonooleat - Polyoxyethylen ist; und das Gewebe mit der Tensidlösung über einen Zeitraum zwischen 6 und ca. 24 h bei einem pH-Wert von 7,0—7,6 kontaktiert wird.

13. Verfahren nach Anspruch 12, wobei das Gewebe während der auf die Fixierung folgenden Steriolisation mit Tensid kontaktiert wird und die Tensidlösung ferner 4—5% Formaldehyd aufweist.

14. Verfahren zum Behandeln von biologischem Gewebe vor der Implantation in ein Tier, um Calcifikation zu hemmen, umfassend die Schritte:

Fixieren des biologischen Gewebes unter Gewebefixierbedingunge; und .

Kontaktieren des fixierten biologischen Gewebes mit einer Lösung eines kationischen Tensids in einer zur Verbinderung der Calcifikation des Gewebes nach der Implantation wirksamen Menge, wobei das kationische Tensid ein Halogenidsalz von Trimethylphenylamin oder Hexadecyltrimethylamin ist.

15. Verfahren nach Anspruch 14, wobei das Tensid Trimethylphenylammoniumchlorid oder Hexadecyltrimethylammoniumbromid ist.

16. Verfahren nach Anspruch 14, wobei das Salz Chlorid oder Bromid ist.

17. Verfahren nach einem der Ansprüche 14—16, wobei das Gewebe mit dem Tensid während der Fixierung kontaktiert wird.

18. Verfahren nach einem der Ansprüche 14—16, wobei das Gewebe mit dem Tensid während der auf die Fixierung folgenden Sterilisation kontaktiert wird.

19. Verfahren nach Anspruch 14, wobei die Tensidmenge in der Lösung 0,1—10 Gew.-% beträgt.

20. Verfahren nach Anspruch 19, wobei die Tensidmenge in der Lösung 0,5—5 Gew.-% beträgt.

21. Verfahren nach Anspruch 19 oder 20, wobei das Gewebe mit der Tensidlösung über einen Zeitraum von 2—30 h kontaktiert wird.

22. Verfahren nach einem der Ansprüche 14—21, wobei das Gewebe mit Glutaraldehyd fixiert wird.

23. Verfahren nach Anspruch 14, wobei das Gewebe mit Glutaraldehyd fixiert und mit einer Lösung kontaktiert wird, die 0,1—10 Gew.-% Tensid enthält, das Trimethylphenylammoniumhalogenid oder Hexadecyltrimethylammoniumhalogenid ist.

24. Verfahren nach einem der Ansprüche 14—23, wobei das biologische Gewebe Sehne, Ligament, Herzklappe, Dura mater oder Herzbeutel ist.

25. Verfahren nach Anspruch 14, wobei die Tensidmenge in der Lösung, 0,1—1,5 Gew.-% beträgt; das biologische Gewebe Sehne, Ligament, Herzklappe, Dura mater oder Herzbeutel und mit Glutaraldehyd fixiert ist; das Tensid Trimethylphenylammoniumhalogenid oder Hexadecyltrimethylammoniumhalogenid ist; und das Gewebe mit der Tensidlösung über einen Zeitraum zwischen 6 und 24 h bei einem pH-Wert von 7,0—7,6 kontaktiert wird.

26. Verfahren nach Anspruch 25, wobei das Gewebe mit Tensid während der auf die Fixierung folgenden Sterilisation kontaktiert wird und die Tensidlösung ferner 4—5% Formaldehyd aufweist.

27. Verfahren zum Behandeln von biologischem Gewebe vor der Implantation in ein Tier, um Calcifikation zu hemmen, umfassend die Schritte:

Fixieren des biologischen Gewebes unter Gewebefixierbedingungen; und

Kontaktieren des fixierten Gewebes mit einer Lösung des Salzes eines anionischen Tensids in einer zur Verminderung von Calcifikation des Gewebes nach der Implantation wirksamen Menge; wobei das Tensid ein Ethylenoxidsulfat eines aliphatischen Alkohols, ein Alkylphenolsulfat, eine Alkansulfonsäure, eine Alkylarylsulfonsäure, eine Gallensäure, eine Fettsäure, eine N - Alkanoylaminosäure, eine N - acylierte Aminosäure, ein sulfatiertes Ethanolamid oder ein sulfonierter Alkylphenylether ist.

28. Verfahren nach Anspruch 27, wobei das Tensid das Natrium-, Kalium- oder Ammoniumsalz ist.

29. Verfahren nach Anspruch 27 oder 28, wobei das Gewebe mit dem Tensid während der Fixierung kontaktiert wird.

30. Verfahren nach Anspruch 27 oder 28, wobei das Gewebe mit dem Tensid während der auf die Fixierung folgenden Sterilisation kontaktiert wird.

31. Verfahren nach einem der Ansprüche 27—30, wobei die Tensidmenge in der Lösung 0,1—10 Gew.-% beträgt.

32. Verfahren nach Anspruch 31, wobei die Tensidmenge in der Lösung 0,5—5 Gew.-% beträgt.

33. Verfahren nach Anspruch 31 oder 32, wobei das Gewebe mit der Tensidlösung über einen Zeitraum von 2—30 h kontaktiert wird.

34. Verfahren nach einem der Ansprüche 27—33, wobei das Gewebe mit Glutaraldehyd fixiert wird.

35. Verfahren nach Anspruch 27, wobei das Gewebe mit Glutaraldehyd fixiert und mit einer Lösung kontaktiert wird, die 0,1—10 Gew.-% Tensid enthält, das ein Ethylenoxidsulfat eines aliphatischen Alkohols, ein Alkylphenolsulfat, eine Alkansulfonsäure oder eine Alkylarylsulfonsäure ist.

36. Verfahren nach einem der Ansprüche 27—35, wobei das biologische Gewebe Sehne, Ligament, Herzklappe, Dura mater oder Herzbeutel ist.

37. Verfahren nach Anspruch 27, wobei die Tensidmenge in der Lösung 0,1—1,5 Gew.-% beträgt; das biologische Gewebe Sehne, Ligament, Herzklappe, Dura mater oder Herzbeutel und mit Glutaraldehyd fixiert ist; das Tensid N-Lauroylsarkosin ist; und das Gewebe mit der Tensidlösung über einen Zeitraum von 6—24 h bei einem pH-Wert von 7,0—7,6 kontaktiert wird.

38. Verfahren nach Anspruch 37, wobei das Gewebe mit Tensid während der auf die Fixierung folgenden Sterilisation kontaktiert wird und die Tensidlösung ferner 4—5% Formaldehyd aufweist.

**Revendications**

1. Procédé de traitement biologique d'un tissu, préalablement à son implantation chez un animal, pour empêcher la calcification, comprenant les étapes de
—fixation du tissu biologique sous des conditions de fixation de tissus; et
—mise en contact du tissu biologique fixé avec une solution d'un agent tensioactif non ionique en quantité suffisante pour réduire la calcification du tissu après l'implantation.

2. Procédé selon la revendication 1, dans lequel l'agent tensioactif est choisi parmi les éthers polyoxyalkylénés, les alkylaryl éthers polyoxyalkylénés, les esters polyoxyalkylénés, les esters de sorbitan et les esters de sorbitan polyoxyalkyléné.

3. Procédé selon la revendication 1 ou 2, dans lequel le tissu est mis en contact avec l'agent tensioactif durant la fixation.

4. Procédé selon la revendication 1 ou 2, dans lequel le tissu fixé est mis en contact durant la stérilisation qui suit la fixation.

5. Procédé selon n'importe laquelle des revendications précédentes dans lequel la quantité d'agent tensioactif dans la solution est de 0,1 à 10% en poids.

6. Procédé selon la revendication 5, dans lequel la quantité d'agent tensioactif dans la solution est de 0,5 à 5% en poids.

7. Procédé selon la revendication 5 ou 6, dans lequel le tissu est mis en contact avec une solution de surfactant pour une durée comprise entre 2 à 30 heures.

8. Procédé selon la revendication 7 dans lequel l'agent tensioactif est du monooléate de sorbitan polyoxyéthyléné.

9. Procédé selon n'importe laquelle des revendications précédentes, dans lequel le tissu est fixé avec de la glutaraldéhyde.

10. Procédé selon la revendication 1, dans lequel le tissu est fixé avec de la glutaraldéhyde, et est mis en contact avec une solution contenant de 0,1 à 10% en poids d'agent tensioactif choisi parmi les éthers polyoxyalkylénés, les alkylaryl éthers polyoxyalkylénés, les esters polyoxyalkylénés, les esters de sorbitan et les esters de sorbitan polyoxyalkyléné.

11. Procédé selon n'importe laquelle des revendications précédentes, dans lequel le tissu biologique est un tendon, un ligament, une valvule cardiaque, une dure-mère ou un péricarde.

12. Procédé selon la revendication 1 dans lequel la quantité d'agent tensioactif dans la solution est comprise entre 0,1 et 1,5% en poids, le tissu biologique est un tendon, un ligament, une valvule cardiaque, une dure-mère, ou un péricarde et est fixé avec de la glutaraldéhyde, l'agent tensioactif est le monooléate de polyoxyethylène, et le tissu est mis en contact avec la solution d'agent tensioactif pour une durée comprise entre 6 heures et environ 24 heures à un pH compris entre 7,0 à 7,6.

13. Procédé selon la revendication 12, dans lequel le tissu est mis en contact avec la solution d'agent tensioactif durant la stérilisation qui suit la fixation et la solution d'agent tensioactif contient de plus 4 à 5% de formaldéhyde.

14. Procédé de traitement d'un tissu biologique antérieurement à son implantation chez un animal pour empêcher la calcification, comprenant les etapes suivantes:
—fixation du tissu biologique sous des conditions de fixation de tissu, et
—mise en contact du tissu biologique fixé avec une solution d'un agent tensioactif cationique en quantité efficace pour réduire la calcification du tissu après implantation, dans lequel l'agent tensioactif cationique est choisi parmi les halogénures de sels de triméthylphénylamine et d'hexadécyltriméthylamine.

15. Procédé selon la revendication 14 dans lequel l'agent tensioactif est choisi parmi le chlorure de trimethylphénylammonium et le bromure de hexadécyltriméthylammonium.

16. Procédé selon la revendication 14, dans lequel le sel est un bromure ou un chlorure.

17. Procédé selon n'importe laquelle des revendications 14 à 16, dans lequel le tissu est mist en contact avec l'agent tensioactif durant la fixation.

18. Procédé selon n'importe laquelle des revendications 14 à 16, dans lequel le tissu est mis en contact avec l'agent tensioactif durant la stérilisation qui suit la fixation.

19. Procédé selon la revendication 14, dans lequel la quantité d'agent tensioactif dans la solution est de 0,1 à 10% en poids.

20. Procédé selon la revendication 19, dans lequel la quantité d'agent tensioactif dans la solution est de 0,5 à 5% en poids.

21. Procédé selon la revendication 19 ou 20, dans lequel le tissu est mis en contact avec la solution d'agent tensioactif pour une durée de 2 à 30 heures.

22. Procédé selon n'importe laquelle des revendications 14 à 21, dans lequel le tissue est fixé avec de la glutaraldéhyde.

23. Procédé selon la revendication 14, dans lequel le tissu est fixé avec de la glutaraldéhyde, et est mis en contact avec une solution contenant de 0,1 à 10% en poids d'un agent tensioactif choisi parmi les halogénures de triméthylphénylammonium et les halogénures d'hexadécyltriméthylammonium.

24. Procédé selon n'importe laquelle des revendications 14 à 23, dans lequel le tissu biologique est un tendon, un ligament, une valvule cardiaque, une dure-mère ou un péricarde.

25. Procédé selon la revendication 14, dans lequel la quantité d'agent tensioactif dans la solution est de 0,1 à 1,5% en poids, le tissu biologique est un tendon, un ligament, une valvule cardiaque, une dure-mère ou un péricarde et est fixé avec de la glutaraldéhyde, l'agent tensioactif est choisi parmi les halogénures de triméthylphénylammonium et les halogénures d'hexadécyltriméthylammonium; et le tissu est mis en contact avec la solution d'agent tensioactif pour une période de 6 à 24 heures à un pH de 7,0 à 7,6.

26. Procédé selon la revendication 25, dans lequel le tissu est mis en contact durant la stérilisation qui suit la fixation et le la solution d'agent tensioactif comprend, de plus, de 4 à 5% de formaldéhyde.

27. Procédé de traitement d'un tissu biologique antérieurement à son implantation chez un animal pour empêcher la calcification, comprenant les étapes suivantes:

—fixation du tissu biologique sous des conditions de fixation de tissu, et

—mise en contact du tissu biologique fixé avec une solution d'un agent tensioactif anionique en quantité efficace pour réduire la calcification du tissu après implantation, dans lequel l'agent tensioactif anionique est choisi parmi les sulfates oxyéthylénés d'alcools aliphatiques, les sulfates d'alkylphénols, les acides alkylsulfoniques, les acides alkylarylsulfoniques, les acides biliaires, les acides gras, les acides aminés N-alkanoyle, les acides aminés N-acylés, les éthanol amides sulfatés et les éthers alkylphényl sulfones.

28. Procédé selon la revendication 27, dans lequel l'agent tensioactif est un sel de sodium, de potassium ou d'ammonium.

29. Procédé selon la revendication 27, dans lequel le tissu est mis en contact avec l'agent tensioactif durant la fixation.

30. Procédé selon la revendication 27 ou 28, dans lequel le tissu est mis en contact avec l'agent tensioactif durant la stérilisation qui suit la fixation.

31. Procédé selon n'importe laquelle des revendications 27 à 30, dans lequel la quantité d'agent tensioactif dans la solution est de 0,1 à 10% en poids.

32. Procédé selon la revendication 31, dans lequel la quantité d'agent tensioactif dans la solution est de 0,5 à 5% en poids.

33. Procédé selon la revendication 31 ou 32, dans lequel le tissu est mis en contact avec une solution d'agent tensioactif pour une durée de 2 à 30 heures.

34. Procédé selon n'importe laquelle des revendications 27 à 33 dans lequel le tissu est fixé avec de la glutaraldéhyde.

35. Procédé selon la revendication 27, dans lequel le tissu est fixé avec de la glutaraldéhyde, et est mis en contact avec une solution contenant de 0,1 à 10% en poids d'agent tensioactif choisi parmi les sulfates oxyéthyléné d'alcools aliphatiques, les sulfates d'alkylphénols, les acides alkylsulfoniques et les acides alkylarylsulfoniques.

36. Procédé selon n'importe laquelle des revendications 27 à 35, dans lequel le tissu biologique est un tendon, un ligament, une valvule cardiaque, une dure-mère ou un péricarde.

37. Procédé selon la revendication 27, dans lequel la quantité d'agent tensioactif dans la solution est de 0,1 à 1,5% en poids, le tissu biologique est un tendon, un ligament, une valvule cardiaque, une dure-mère ou un péricarde et est fixé avec de la glutaraldéhyde, l'agent tensioactif est la N-lauroylsarcosine, et le tissu est mis en contact avec la solution d'agent tensioactif pour une durée de 6 à 24 heures à un pH de 7,0 à 7,6.

38. Procédé selon la revendication 37, dans lequel le tissu est mis en contact avec l'agent tensioactif pendant la stérilisation qui suit la fixation et la solution d'agent tensioactif comprend, de plus, de 4 à 5% de formaldéhyde.